(19) Europäisches Patentamt
European Patent Office
Office européen des brevets

(11) EP 2 960 319 B1

(12) **EUROPEAN PATENT SPECIFICATION**

(45) Date of publication and mention of the grant of the patent:
**13.03.2019 Bulletin 2019/11**

(51) Int Cl.:
***C10L 1/222*** *(2006.01)* ***C10L 1/224*** *(2006.01)*

(21) Application number: **15172000.0**

(22) Date of filing: **12.06.2015**

(54) **HYDROCARBYL SOLUBLE QUATERNARY AMMONIUM CARBOXYLATES AND FUEL COMPOSITIONS CONTAINING THEM**

KOHLENWASSERSTOFFLÖSLICHE QUATERNÄRE AMMONIUMCARBOXYLATE UND BRENNSTOFFZUSAMMENSETZUNGEN DAMIT

CARBOXYLATES D'AMMONIUM QUATERNAIRE SOLUBLE HYDROCARBYLE ET COMPOSITIONS DE CARBURANT CONTENANT CES DERNIERS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2014 US 201414315302**

(43) Date of publication of application:
**30.12.2015 Bulletin 2015/53**

(73) Proprietor: **Afton Chemical Corporation**
**Richmond, Virginia 23219 (US)**

(72) Inventors:
- **Fang, Xinggao**
  **Midlothian, VA 23114 (US)**
- **Schwab, Scott D.**
  **Richmond, VA 23233 (US)**
- **Taylor, Daniel**
  **Fredericksburg, VA 22405 (US)**

(74) Representative: **BRP Renaud & Partner mbB**
**Rechtsanwälte Patentanwälte**
**Steuerberater**
**Königstraße 28**
**70173 Stuttgart (DE)**

(56) References cited:
**EP-A1- 0 293 192**
**WO-A1-2010/101801**
**WO-A1-2013/070503**
**US-A- 3 158 647**
**US-A1- 2008 307 698**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 960 319 B1

## Description

### TECHNICAL FIELD:

The disclosure is directed to a fuel additive compositions and to fuels that include the additive composition that are useful for improving the performance of fuel injected engines, reducing engine wear, improving fuel demulsibility. In particular the disclosure is directed to fuel additive compositions that include hydrocarbyl soluble quaternary ammonium carboxylates and to methods for using the carboxylates in a fuel composition.

### BACKGROUND AND SUMMARY:

Fuel compositions for vehicles are continually being improved to enhance various properties of the fuels in order to accommodate their use in newer, more advanced engines. Accordingly, the fuel compositions contain additives which are directed to certain properties that require improvement. For example, friction modifiers, such as fatty acid amides, are added to fuel to reduce friction and wear in the fuel delivery systems of an engine. Other additives are included in the fuel compositions to reduce the corrosion potential of the fuel composition and/or improve the conductivity property of the fuel composition. Still other additives are added to the fuel to improve the fuel economy of an engine operating on the fuel. Each of the foregoing additives may be effective to improve a single property of the fuel composition and, in some instances, may adversely affect other properties of the fuel composition. Accordingly, fuel compositions typically include a complex mixture of additives that are selected to cooperate with each other to improve the fuel composition. Some of the additives may be beneficial for one characteristic, but detrimental to another characteristic of the fuel. Accordingly, there is a need for a fuel additive that is effective to improve multiple characteristics of a fuel.

Engine and fuel delivery system deposit is a particularly important problem for modern combustion engines and deposit control additives are used to mitigate this problem. For example, diesel engines suffer deposit in the fuel delivery system. Well known succinimide type detergents offer limited detergency as measured by industry DW10 and XUD9 tests.

Gasoline engines also suffer deposit problems. Commonly known type detergent such as Mannich detergent did not provide sufficient cleaning power.

Quaternary ammonium compounds such as alkoxylated salts have recently been developed as very effective detergents compared to conventional succinimide and Mannich base detergents. Quaternary ammonium compounds are known as is disclosed in US Patent No. 8,147,569. However highly dangerous ethylene oxides and propylene oxides are required to make such detergents.

Quaternary ammonium compounds through alkylation with dialkyl carbonate are also disclosed in US Patent No. 8,147,569. However the carbonate anion part of the molecule is susceptible to precipitation and drop out in fuels or additive packages. In addition, the detergency of quaternary ammonium carbonates may still need to be improved.

EP 0 293 19 2 A1 teaches a diesel fuel composition containing a diesel fuel and a minor proportion by weight of a quaternary ammonium salt soluble therein. The quaternary ammonium salt is described as being effective in reducing fouling of injector nozzles and in improving combustion chamber and piston cleanliness of diesel engines. The quaternary ammonium salt includes dicocodimethyl ammonium formate and dicocodimethyl ammonium oxalate.

WO 2013/070503A1 teaches a fuel composition for a direct fuel injected gasoline engine comprising, a method for improving performance of fuel injectors and a method for cleaning fuel injectors for an internal combustion gasoline engine. The fuel composition includes a major amount of fuel and a minor, effective amount of a quaternary ammonium salt having a thermogravimetric analysis (TGA) weight loss of greater than 50 wt.% at 350° C. The amount of quaternary ammonium salt present in the fuel is sufficient to improve performance of the direct fuel injected engine having combusted the composition compared to the performance of such engine having combusted a fuel composition that does not contain the quaternary ammonium salt. In an example, this reference teaches that the quaternary ammonium salt is formed in a reaction between an oleylamido propyldimethylamine and $C_{14}$-methyl salicylate.

The invention is defined in the appended claims. In accordance with the disclosure, exemplary embodiments provide a fuel additive composition, fuel composition, method of improving the injector performance of a fuel injected engine, and a method of reducing wear in a fuel system of an engine. The fuel composition includes from 5 to 300 ppm by weight based on a total weight of the fuel composition of a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-y-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid, and wherein the quaternary ammonium carbonate is formed by reacting a carbonic acid diester with a tertiary amido amine compound of the following formula

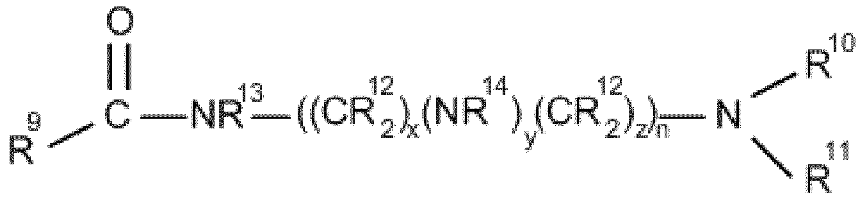

wherein each of $R^{10}$, and $R^{11}$ is selected from hydrocarbyl groups containing from 1 to 200 carbon atoms, each $R^9$, $R^{12}$, $R^{13}$ and $R^{14}$ is independently selected from hydrogen or a hydrocarbyl group, x is 1 to 6, y is 0 or 1, z is 1 to 6, and n is 1 to 6; and wherein the hydrocarbyl groups have a carbon atom directly attached to a remainder of the molecule and each hydrocarbyl group is independently selected from hydrocarbon substituents, and substituted hydrocarbon substituents containing one or more halo groups, hydroxy groups, alkoxy groups, mercapto groups, nitro groups, nitroso groups, amino groups, sulfoxy groups, pyridyl groups, furyl groups, thienyl groups, imidazolyl groups, sulfur, oxygen, and nitrogen, and wherein no more than two non-hydrocarbon substituents are present for every ten carbon atoms in the hydrocarbyl group.

One embodiment of the disclosure provides a method of improving the injector performance of a fuel injected engine. The method includes combusting in the engine a fuel composition comprising a major amount of fuel and from 5 to 300 ppm by weight based on a total weight of the fuel composition of a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-y-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid.

Another embodiment of the disclosure provides a method of reducing wear in a fuel system of an engine. The method includes operating the engine on a fuel composition comprising a major amount of fuel and from 5 to 300 ppm by weight based on a total weight of the fuel composition of a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-y-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid.

It is further disclosed a method of improving the demulsibility of a fuel composition. The method includes providing as a fuel composition a major amount of fuel and from 5 to 300 ppm by weight based on a total weight of the fuel composition of a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-y-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid.

Another embodiment of the disclosure provides a process of making a hydrocarbyl substituted amido-trialkyl quaternary ammonium carbonate. The process includes reacting an amidodialkylamine with dialkylcarbonate in the mole ratio of amine to carbonate of from 1:1 to 1:1.5 at a temperature ranging from 120° to 160° C in a reaction medium substantially devoid of a protic solvent.

Additives of the disclosure may overcome the deficiencies of current known fuel detergents by providing improved detergency and reduced negative impact on fuel demulsibility. In addition, the additive may also be capable of

reducing engine wear using both petroleum and ethanol containing gasoline fuels.

An advantage of the compositions and methods described herein is that the additive composition and the fuel composition may not only improve the friction and wear properties of the fuel, but the additive composition may be effective to improve fuel economy, and/or to clean up or prevent deposits on engine parts and in fuel systems for engines at relatively low treat rates.

Another advantage of the fuel additive described herein is that the additive composition may be used at a relatively low concentration in combination with conventional fuel additives to provide enhanced engine performance.

Additional details and advantages of the disclosure will be set forth in part in the description which follows, and/or may be learned by practice of the disclosure. The details and advantages of the disclosure may be realized and attained by means of the elements and combinations particularly pointed out in the appended claims.

## DETAILED DESCRIPTION OF EXEMPLARY EMBODIMENTS

The fuel additive component of the present application may be used in a minor amount in a major amount of fuel and may be added to the fuel directly or added as a component of an additive concentrate to the fuel. A particularly suitable fuel additive component for use in methods of improving the operation of internal combustion engines may be made by reacting a tertiary amine of the formula

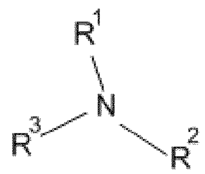

wherein each of $R^1$, $R^2$, and $R^3$ is selected from hydrocarbyl groups containing from 1 to 200 carbon atoms, with a dialkyl carbonate and subsequent reaction of the resulting quaternary ammonium carbonate with an acid to provide a hydrocarbyl soluble quaternary ammonium carboxylate. The quaternary ammonium carbonate may also be derived from a tertiary amido amine and a dialkyl carbonate. Regardless of how the hydrocarbyl quaternary ammonium carbonate is made, a key feature of the disclosure is that the resulting quaternary ammonium carbonate is reacted with an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-y-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid to provide the hydrocarbyl soluble quaternary ammonium carboxylate. The quaternary ammonium carboxylate of the present invention is hydrocarbyl soluble, i.e., at least 0.1 grams of the quaternary ammonium carboxylate will dissolve in 100 grams of heptane at room temperature upon stirring, preferably at least 5 grams of the quaternary ammonium carboxylate will dissolve in 100 grams of heptane at room temperature upon stirring.

As used herein, the term "hydrocarbyl group" or "hydrocarbyl" is used in its ordinary sense, which is well-known to those skilled in the art. Specifically, it refers to a group having a carbon atom directly attached to the remainder of a molecule and having a predominantly hydrocarbon character. Examples of hydrocarbyl groups include:

(1) hydrocarbon substituents, that is, aliphatic (e.g., alkyl or alkenyl), alicyclic (e.g., cycloalkyl, cycloalkenyl) substituents, and aromatic-, aliphatic-, and alicyclic-substituted aromatic substituents, as well as cyclic substituents wherein the ring is completed through another portion of the molecule (e.g., two substituents together form an alicyclic radical);

(2) substituted hydrocarbon substituents, that is, substituents containing non-hydrocarbon groups which, in the context of the description herein, do not alter the predominantly hydrocarbon substituent (e.g., halo (especially chloro and fluoro), hydroxy, alkoxy, mercapto, alkylmercapto, nitro, nitroso, amino, alkylamino, and sulfoxy);

(3) hetero-substituents, that is, substituents which, while having a predominantly hydrocarbon character, in the context of this description, contain other than carbon in a ring or chain otherwise composed of carbon atoms. Heteroatoms include sulfur, oxygen, nitrogen, and encompass substituents such as pyridyl, furyl, thienyl, and imidazolyl. In general, no more than two, or as a further example, no more than one, non-hydrocarbon substituent will be present for every ten carbon atoms in the hydrocarbyl group; in some embodiments, there will be no non-hydrocarbon substituent in the hydrocarbyl group.

The term "alkyl" as employed herein refers to straight, branched, cyclic, and/or substituted saturated chain moieties of from 1 to 100 carbon atoms.

The term "alkenyl" as employed herein refers to straight, branched, cyclic, and/or substituted unsaturated chain moieties of from 3 to 100 carbon atoms.

The term "aryl" as employed herein refers to single and multi-ring aromatic compounds that may include alkyl, alkenyl, alkylaryl, amino, hydroxyl, alkoxy, halo substituents, and/or heteroatoms including, but not limited to, nitrogen, oxygen, and sulfur.

As used herein, the term "major amount" is understood to mean an amount greater than or equal to 50 wt. %, for example from 80 to 98 wt.% relative to the total weight of the composition. Moreover, as used herein, the term "minor amount" is understood to mean an amount less than 50 wt. % relative to the total weight of the composition.

Amine Compound

In one embodiment, a tertiary amine including diamines and polyamines may be reacted with a $C_1$ to $C_{54}$ carboxylic acid to form an amido amine and the amido amine may be subsequently reacted with a quaternizing agent. Suitable tertiary amido amine compounds may have a hydrocarbyl linkage, such as an ether linkage between the amido group and the amino group or the tertiary amido amine may be a compound of the formula

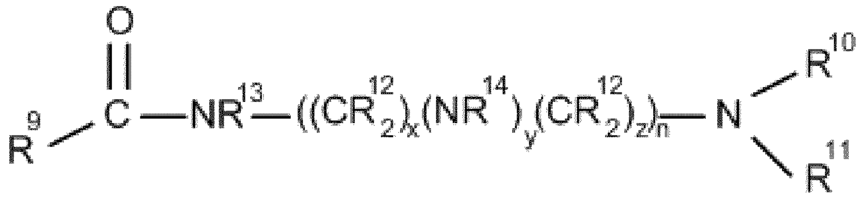

may be used, wherein each of $R^{10}$, and $R^{11}$ is selected from hydrocarbyl groups containing from 1 to 200 carbon atoms, preferably 1 to 25 carbon atoms, more preferably 1 to 8 carbon atoms, and most preferably 1 to 2 carbon atoms, each $R^9$, $R^{12}$, $R^{13}$ and $R^{14}$ may be independently selected from hydrogen or a hydrocarbyl group wherein $R^9$ preferably is a hydrocarbyl having 5 to 100 carbon atoms, more preferably 11 to 20 carbon atoms, x may range from 1 to 6, y may be 0 or 1, z may be 1 to 6, and n may range from 1 to 6. Each hydrocarbyl group $R^9$ to $R^{14}$ may independently be linear, branched, substituted, cyclic, saturated, unsaturated, or contain one or more hetero atoms. Suitable hydrocarbyl groups may include, but are not limited to alkyl groups, aryl groups, alkylaryl groups, arylalkyl groups, alkoxy groups, aryloxy groups, amino groups, and the like. Particularly suitable hydrocarbyl groups may be linear or branched alkyl groups. A representative example of an amine reactant which may be amidized and quaternized to yield compounds disclosed herein include for example, but are not limited to, dimethyl amino propyl amine and 2-(2-dimethylamino-ethoxy) ethylamine.

If the amine contains solely primary or secondary amino groups, it may be desirable to alkylate at least one of the primary or secondary amino groups to a tertiary amino group prior to quaternizing. In one embodiment, alkylation of primary amines and secondary amines or mixtures with tertiary amines may be exhaustively or partially alkylated to a tertiary amine, and then converted into a quaternary ammonium carbonate salt.

When the amine has a hydroxyl group, the amine may be converted to an ester amine by reacting the hydroxyl group of the amine with a $C_1$ to $C_{54}$ carboxylic acid. The acid may be a monoacid, a dimer acid, or a trimer acid. The acid may be selected from the group consisting of formic acid, acetic acid, propionic acid, butyric acid, caprylic acid, capric acid, lauric acid, myristic acid, palmitic acid, stearic, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid, docosahexaenoic acid, and the dimer and trimer acids thereof. When reacted with the amine, the reaction product may be a $C_1$-$C_{54}$-alkyl or alkenyl-substituted ester amine such as a $C_1$-$C_{54}$-alkyl or alkenyl-substituted ester propyldimethylamine.

In another embodiment for use in the methods of the invention, the tertiary amine may be a reaction product of a hydrocarbyl substituted succinic anhydride and a tertiary amine of the formula

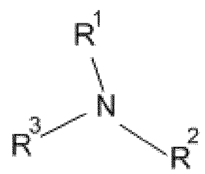

wherein $R^1$, $R^2$, and $R^3$ are defined as above. Suitable tertiary amines include, but are not limited to 1-aminopiperidine, 1-(2-aminoethyl)piperidine, 1-(3-aminopropyl)-2-pipecoline, 1-methyl-(4-methylamino)piperidine, 4-(1-pyrrolidinyl)piperidine, 1-(2-aminoethyl)pyrrolidine, 2-(2-aminoethyl)-1-methylpyrrolidine, N,N-diethylethylenediamine, N,N-dimethylethy-lenediamine, N,N-dibutylethylenediamine, N,N-diethyl-1,3-diaminopropane, N,N-dimethyl-1,3-diaminopropane, N,N,N'-trimethylethylenediamine, N,N-dimethyl-N'-ethylethylenediamine, N,N-diethyl-N'-methylethylenediamine, N,N,N'-triethylethylenediamine, 3-dimethylamino-propylamine, 3-diethylaminopropylamine, 3-dibutylaminopropylamine, N,N,N'-trimethyl-1,3- propanediamine, N,N,2,2-tetramethyl-1,3-propanediamine, 2-amino-5-diethylaminopentane, N,N,N',N'-tetraethyldiethylenetriamine, 3,3'-diamino-N-methyldipropylamine, 3,3'-iminobis-(N,N-dimethylpropylamine), 1-(3-aminopropyl)imidazole, 4-(3-aminopropyl)morpholine, 1-(2- aminoethyl)piperidine, 3,3-diamino-N-methyldipropylamine, 3,3-aminobis(N,N-dimethylpropyl- amine), N,N,N'-trimethyl-N'-hydroxyethyl bisaminoethyl ether, N,N-bis(3-dimethylamino-propyl)-N-isopropanolamine, bis(N,Ndimethylaminopropyl)amine, 2-(2-dimethylaminoethoxy-ethanol, 2-dimethylaminoethyl methyl ethanolamine, or combinations thereof.

Other suitable tertiary amines may include alkanolamines such as triethanolamine, N,N-dimethylaminopropanol, N,N-diethylaminopropanol, N,N-diethylaminobutanol, triisopro-panolamine, 1-[2-hydroxyethyl]piperidine, 2-[2-(dimethylamine)ethoxy]-ethanol, N-ethyldi-ethanolamine, N-methyldiethanolamine, N-butyldiethanolamine, N,N-diethylaminoethanol, N,N-dimethyl amino- ethanol, 2-dimethylamino-2-methyl-1-propanol, N,N,N'-trimethyl-N'-hydroxyethyl bisaminoethyl ether, N,N-bis(3-dimethylaminopropyl)-N-isopropanolamine, bis(N,Ndimethylaminopropyl)amine, 2-(2-dimethylaminoethoxy-ethanol, 2-dimethylaminoethyl methyl ethanolamine, or combinations thereof. Other amines that may be used include Mannich base amines and ether or carbonyl capped Mannich base amines.

Any of the foregoing tertiary amines may be reacted with a hydrocarbyl substituted acylating agent that may be selected from a hydrocarbyl substituted mono- di- or polycarboxylic acid or a reactive equivalent thereof. A particularly suitable acylating agent is a hydrocarbyl substituted succinic acid, ester, anhydride, mono-acid/mono-ester, or diacid.

Quaternizing Agent

A suitable quaternizing agent may be selected from a carbonic acid diester, such as dimethyl carbonate, ethylmethyl carbonate, diethyl carbonate, di-propyl carbonate, dibutyl carbonate, cyclic carbonates, and the like. A particularly suitable carbonic acid diester may be selected from dimethyl carbonate and diethylcarbonate. The reaction between the tertiary amine and carbonate may be carried out by contacting and mixing the amine with the carbonate in the reaction vessel in the absence of alcohol.

The reaction may be carried out at temperature ranging from 100° to 200° C, for example from 110° to 170° C. The reaction may be conducted by reacting any amount of tertiary amino groups to carbonate groups sufficient to provide a quaternary ammonium compound. In one embodiment a mole ratio of tertiary amino groups to carbonate may range from 2:1 to 1:5, or from 1:1 to 1:2, or from 1:1 to 1:1.5. The reaction may optionally be conducted in the presence of alcohol or water and excess of dialkyl carbonate. Contrary to the prior art teaching it was surprisingly found that for certain amido amines, a high yield of quaternary ammonium salt may be achieved by reacting in the absence of alcohol or water solvents and limited amounts of dialkyl carbonate. When the reaction is completed volatiles and unreacted reagents may be removed from the reaction product by heating the reaction product under vacuum. The product may be diluted with mineral oil, diesel fuel, kerosene, alcohol, or an inert hydrocarbon solvent to prevent the product from being too viscous, if necessary.

The resulting quaternary ammonium carbonate compound is then reacted with an organic acid to provide the hydrocarbyl soluble quaternary ammonium carboxylate. Specific examples of the organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-yl-7,1 -octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid. The amount of acid reacted with the quaternary ammonium carbonate may range from 10:1 to 1:10, for example 0.5:1 to 2:1, or from 0.8:1 to 1.5:1 equivalents of acid per equivalent of carbonate.

One or more additional optional compounds may be present in the fuel compositions of the disclosed embodiments. For example, the fuels may contain conventional quantities of cetane improvers, octane improvers, corrosion inhibitors, cold flow improvers (CFPP additive), pour point depressants, solvents, demulsifiers, lubricity additives, friction modifiers, amine stabilizers, combustion improvers, detergents, dispersants, antioxidants, heat stabilizers, conductivity improvers, metal deactivators, marker dyes, organic nitrate ignition accelerators, cyclomatic manganese tricarbonyl compounds, carrier fluids, and the like. In some aspects, the compositions described herein may contain 10 weight percent or less, or in other aspects, 5 weight percent or less, based on the total weight of the additive concentrate, of

one or more of the above additives. Similarly, the fuels may contain suitable amounts of conventional fuel blending components such as methanol, ethanol, dialkyl ethers, 2-ethylhexanol, and the like.

When formulating the fuel compositions of this application, the additives may be employed in amounts sufficient to reduce or inhibit deposit formation in a fuel system or combustion chamber of an engine and/or crankcase. In some aspects, the fuels may contain minor amounts of the above described reaction product that controls or reduces the formation of engine deposits, for example injector deposits in engines. For example, the fuels of this disclosure may contain, on an active ingredient basis, an amount of the quaternary ammonium carboxylate in the range of 1 mg to 300 mg of quaternary ammonium carboxylate per Kg of fuel, such as in the range of 5 mg to 200 mg of per Kg of fuel or in the range of from 10 mg to 100 mg of the quaternary ammonium carboxylate per Kg of fuel. The active ingredient basis excludes the weight of (i) unreacted components associated with and remaining in the product as produced and used, and (ii) solvent(s), if any, used in the manufacture of the product either during or after its formation.

The additives of the present application, including the quaternary ammonium carboxylate described above, and optional additives used in formulating the fuels of this invention may be blended into the base fuel individually or in various sub-combinations. In some embodiments, the additive components of the present application may be blended into the fuel concurrently using an additive concentrate, as this takes advantage of the mutual compatibility and convenience afforded by the combination of ingredients when in the form of an additive concentrate. Also, use of a concentrate may reduce blending time and lessen the possibility of blending errors.

The fuels of the present application may be applicable to the operation of diesel, jet, or gasoline engines. The engine include both stationary engines (e.g., engines used in electrical power generation installations, in pumping stations, etc.) and ambulatory engines (e.g., engines used as prime movers in automobiles, trucks, road-grading equipment, military vehicles, etc.). For example, the fuels may include any and all middle distillate fuels, diesel fuels, biorenewable fuels, biodiesel fuel, fatty acid alkyl ester, gas-to-liquid (GTL) fuels, gasoline, jet fuel, alcohols, ethers, kerosene, low sulfur fuels, synthetic fuels, such as Fischer-Tropsch fuels, liquid petroleum gas, bunker oils, coal to liquid (CTL) fuels, biomass to liquid (BTL) fuels, high asphaltene fuels, fuels derived from coal (natural, cleaned, and petcoke), genetically engineered biofuels and crops and extracts therefrom, and natural gas. "Biorenewable fuels" as used herein is understood to mean any fuel which is derived from resources other than petroleum. Such resources include, but are not limited to, corn, maize, soybeans and other crops; grasses, such as switchgrass, miscanthus, and hybrid grasses; algae, seaweed, vegetable oils; natural fats; and mixtures thereof. In an aspect, the biorenewable fuel can comprise monohydroxy alcohols, such as those comprising from 1 to 5 carbon atoms. Non-limiting examples of suitable monohydroxy alcohols include methanol, ethanol, propanol, n-butanol, isobutanol, t-butyl alcohol, amyl alcohol, and isoamyl alcohol.

Accordingly, aspects of the present application are directed to methods for reducing friction or wear in an internal combustion engine or fuel system for an internal combustion engine as well as for reducing a corrosion potential for the fuel in the engine, fuel system or fuel terminal. In another aspect, the quaternary ammonium carboxylate compounds described herein or fuel containing the quaternary ammonium carboxylates may be combined with polyhydrocarbyl-succinimides, polyhydrocarbyl succinic-acids, polyhydrocarbyl-succinamides, polyhydrocarbyl-succinic esters, polyhydrocarbyl-succinic amide/acids and polyhydrocarbyl-succinic acid/esters, reaction products of polyhydrocarbyl succinic anhydride and aminoguanidine and its salts, and Mannich compounds.

In some aspects, the methods comprise injecting a hydrocarbon-based fuel comprising a quaternary ammonium carboxylate of the present disclosure through the injectors of the engine into the combustion chamber, and igniting the fuel. In some aspects, the method may also comprise mixing into the fuel at least one of the optional additional ingredients described above.

## EXAMPLES

The following examples are illustrative of exemplary embodiments of the disclosure. In these examples as well as elsewhere in this application, all parts and percentages are by weight unless otherwise indicated. It is intended that these examples are being presented for the purpose of illustration only and are not intended to limit the scope of the invention disclosed herein.

### Carbonate Example 1

Polyisobutenylsuccinimidopropyl trimethyl ammonium methylcarbonate was prepared according to the procedure of example 4 of U.S. Patent No. 8,147,569.

### Carbonate Example 2

Method A: Oleylamidopropyldimethylamine (125 grams) and dimethyl carbonate (123.1 grams) were charged into a 0.5 L stainless steel pressure reactor with an overhead stirrer at room temperature. The reactor was purged with nitrogen and then heated to 140° C. The reaction mixture was held at 140° C for 6.5 hours and then cooled to a quaternary ammonium carbonate give product as a brownish liquid.

Method B: Oleylamidopropyldimethylamine (190 grams) and dimethyl carbonate (70 grams) were charged into a 0.5 L stainless steel pressure reactor with an overhead stirrer at room temperature. The reactor was purged with nitrogen and then heated to 140° C. The mixture was held at 140° C for 4 hours and then cooled to room temperature. 2-Ethylhexanol (31.5 grams) was added to the mixture to give quaternary ammonium carbonate product as brownish oil.

Method C: Oleylamidopropyldimethylamine (253 grams) and dimethyl carbonate (83 grams) were charged into a 0.5 L stainless steel pressure reactor with an overhead stirrer at room temperature. The reactor was purged with nitrogen and then heated to 140° C. The mixture was held at 140° C for 4 hours and then cooled to room temperature. 2-Ethylhexanol (31.5 grams) was added to the mixture to give quaternary ammonium carbonate product as brownish oil.

**Carbonate Example 3**

$C_{22}$-alkenyl succinimidopropyl dimethyl amine (which was prepared by reacting $C_{22}$-alkenylsuccinic anhydride with dimethylamino propylamine at elevated temperature to remove water) (180 grams), dimethyl carbonate (49 grams), and methanol (57.3 grams) were reacted at 140° C in a stainless steel reactor for 6.5 hours. The resulting quaternary ammonium carbonate product was a brownish oil.

**Inventive Example 1**

To a round bottom flask with overhead stirrer was added oleic acid (46.4 grams) and 2-ethylhexanol (22 grams). About 1 equivalent of quaternary ammonium carbonate compound of Carbonate Example 2 was added dropwise at room temperature while the mixture was being stirred. Gas was generated during the addition of the carbonate compound. The mixture was stirred at room temperature for 4 more hours. Volatiles were then removed under rotary evaporation (90° C, 30 torr) to give a carboxylate product as a brownish oil.

**Inventive Example 2**

A hydrocarbyl soluble quaternary ammonium carboxylate product was made according to Inventive Example 1 except that oleic acid was replaced with 1000 $M_w$ polyisobutenylsuccinic mono methyl ester mono acid. The product was a viscous oil.

**Inventive Example 3**

A hydrocarbyl soluble quaternary ammonium carboxylate product was made according to Inventive Example 1 except that oleic acid was replaced with 1000 $M_w$ polyisobutenylsuccinic mono 2-ethyhexyl ester mono acid.

**Inventive Example 4**

A hydrocarbyl soluble quaternary ammonium carboxylate product was made according to Inventive Example 1 except that oleic acid was replaced with 1000 $M_w$ polyisobutenylsuccinic mono 4-methylpiperazinyl amide mono acid.

**Inventive Example 5**

A hydrocarbyl soluble quaternary ammonium carboxylate product was made according to Inventive Example 1 except that oleic acid was replaced with 1000 MW polyisobutenylsuccinic mono dimethylaminoethyl ester mono acid.

**Inventive Example 6**

A hydrocarbyl soluble quaternary ammonium carboxylate product was made according to Inventive Example 3 except that the quaternary ammonium carbonate product of Carbonate Example 3 was used.

**Inventive Example 7**

A hydrocarbyl soluble quaternary ammonium carboxylate product was made according to Inventive Example 6 except that acid was replaced with 1000 MW polyisobutenylsuccinic mono dimethylaminoethyl ester mono acid.

**Inventive Example 8**

A quaternary ammonium carbonate product was made according to Carbonate Example 3 except that the amine was replaced with dimethyl tridecyloxo-methylethyloxo-methylethyl amine to give an alkylether trimethyl quaternary ammonium compound. The hydrocarbyl soluble carboxylate product was then made according to Inventive Example 6 using the foregoing quaternary ammonium carbonate product instead of the carbonate product of Carbonate Example 3.

**Example 9 (Not according to the invention)**

To a 1000 $M_w$ polyisobutenylsuccinic diacid (200 grams, 78 wt.% active in aromatic solvent 150) and toluene (100 grams) in a round bottom flask with an overhead stirrer was added dropwise a solution of didecyldimethyl ammonium carbonate in water (50 wt.% active, 156 grams). Water and toluene were removed under rotary evaporation (85° C, 20 torr) to give a carboxylate product as a brown oil.

Diesel Engine Test protocol

A DW10 test that was developed by Coordinating European Council (CEC) was used to demonstrate the propensity of fuels to provoke fuel injector fouling and was also used to demonstrate the ability of certain fuel additives to prevent or control these deposits. Additive evaluations used the protocol of CEC F-98-08 for direct injection, common rail diesel engine nozzle coking tests. An engine dynamometer test stand was used for the installation of the Peugeot DW10 diesel engine for running the injector coking tests. The engine was a 2.0 liter engine having four cylinders. Each combustion chamber had four valves and the fuel injectors were DI piezo injectors have a Euro V classification.

The core protocol procedure consisted of running the engine through a cycle for 8-hours and allowing the engine to soak (engine off) for a prescribed amount of time. The foregoing sequence was repeated four times. At the end of each hour, a power measurement was taken of the engine while the engine was operating at rated conditions. The injector fouling propensity of the fuel was characterized by a difference in observed rated power between the beginning and the end of the test cycle.

Test preparation involved flushing the previous test's fuel from the engine prior to removing the injectors. The test injectors were inspected, cleaned, and reinstalled in the engine. If new injectors were selected, the new injectors were put through a 16-hour break-in cycle. Next, the engine was started using the desired test cycle program. Once the engine was warmed up, power was measured at 4000 RPM and full load to check for full power restoration after cleaning the injectors. If the power measurements were within specification, the test cycle was initiated. The following Table 1 provides a representation of the DW10 coking cycle that was used to evaluate the fuel additives according to the disclosure.

**Table 1 - One hour representation of DW10 coking cycle.**

| Step | Duration(minutes) | Engine speed (rpm) | Load (%) | Torque(Nm) | Boost air after Intercooler (°C) |
|---|---|---|---|---|---|
| 1 | 2 | 1750 | 20 | 62 | 45 |
| 2 | 7 | 3000 | 60 | 173 | 50 |
| 3 | 2 | 1750 | 20 | 62 | 45 |
| 4 | 7 | 3500 | 80 | 212 | 50 |
| 5 | 2 | 1750 | 20 | 62 | 45 |
| 6 | 10 | 4000 | 100 | * | 50 |
| 7 | 2 | 1250 | 10 | 25 | 43 |
| 8 | 7 | 3000 | 100 | * | 50 |
| 9 | 2 | 1250 | 10 | 25 | 43 |
| 10 | 10 | 2000 | 100 | * | 50 |

(continued)

| Step | Duration(minutes) | Engine speed (rpm) | Load (%) | Torque(Nm) | Boost air after Intercooler (°C) |
|---|---|---|---|---|---|
| 11 | 2 | 1250 | 10 | 25 | 43 |
| 12 | 7 | 4000 | 100 | * | 50 |

Various fuel additives were tested using the foregoing engine test procedure in a diesel fuel containing zinc neodecanoate, 2-ethylhexyl nitrate, and a fatty acid ester friction modifier (base fuel). A "dirty-up" phase consisting of base fuel only with no additive was initiated, followed by a "clean-up" phase consisting of base fuel with additive. All runs were made with 8 hour dirty-up and 8 hour clean-up unless indicated otherwise. The percent power recovery was calculated using the power measurement at end of the "dirty-up" phase and the power measurement at end of the "clean-up" phase. The percent power recovery was determined by the following formula

$$\text{Percent Power recovery} = (DU-CU)/DU \times 100$$

wherein DU is a percent power loss at the end of a dirty-up phase without the additive, CU is the percent power at the end of a clean-up phase with the fuel additive, and power is measured according to CEC F98-08 DW10 test.

**Table 2 - in Ultra Low Sulfur Diesel Reference Fuel**

| Run | Additives and treat rate (ppm by weight) | Power loss % | | Power recovery% | Additive Efficiency |
|---|---|---|---|---|---|
| | | DU | CU | (DU-CU)/DU X 100 | Power Recovery %/ppm |
| 1 | Carbonate Ex. 1 - (100 ppmw) | -6.22 | -0.78 | 87 | 0.87 |
| 2 | Inventive Ex. 9 (75 ppmw) | -6.31 | 0.26 | 104 | 1.39 |

As shown by the results in the foregoing Table 2, the additive of Inventive Example 9 provided a significant and unexpected improvement in power recovery compared to the quaternary ammonium carbonate product of Carbonate Example 1, even at a 25 wt.% lower treat rate than Carbonate Example 1. In the table, the conventional succinimide detergent is a reaction product of 1000MW PIBSA and tetraethylene pentamine (TEPA) in a mole ratio of about 1.6 to 1 as generally disclosed in US Patent No. 8,475,541.

**Table 3 - in DF-79 Reference Fuel**

| Run | Additives and treat rate (ppm by weight) | Power loss % | | Power recovery% |
|---|---|---|---|---|
| | | DU | CU | (DU-CU)/DU X 100 |
| 1 | Conventional succinimide detergent (85 ppmw) | -4.45 | -3.19 | 28 |
| 2 | Inventive Example 3 - (100 ppmw) | -4.60 | 1.72 | 137 |
| 3 | Inventive Example 4 - (100 ppmw) | -6.75 | 0.22 | 103 |
| 4 | Inventive Example 3 - (50 ppmw) plus conventional succinimide detergent (50 ppmw) | -4.56 | 1.74 | 138 |
| 5 | Inventive Example 3 - (25 ppmw) plus conventional succinimide detergent (75 ppmw) | -5.23 | 0.49 | 109 |
| 6 | Inventive Example 3 - (25 ppmw) | -3.19 | -2.09 | 34 |
| 7 | Inventive Example 7- (50 ppmw) | -4.82 | -2.2 | 54 |
| 8 | Inventive Example 7 - (50 ppmw) plus conventional succinimide detergent (50 ppmw) | -2.2 | -0.09 | 96 |

(continued)

| Run | Additives and treat rate (ppm by weight) | Power loss % | | Power recovery% |
|---|---|---|---|---|
| | | DU | CU | (DU-CU)/DU X 100 |
| 9 | Inventive Example 5 - (50 ppmw) plus conventional succinimide detergent (50 ppmw) | -5.45 | -0.2 | 96 |

Table 3 illustrated the fact that additives according to the disclosure are substantially more effective in increasing the power recovery, even at a lower treat rate, than a conventional succinimide detergent (Runs 6 and 7 compared to Run 1). Inventive Example 3 provided the greatest power recovery either alone (Run 2) or in combination with a convention succinimide detergent (Runs 4 and 5). All of the inventive examples, either alone (Runs 2, 3, 6 and 7) or in combination with a conventional succinimide detergent (Runs 4, 5, 8, and 9 provided an unexpected improvement in power recovery compared to a conventional succinimide detergent (Run 1). The foregoing runs also demonstrated an unexpected synergistic effect between the conventional detergent (Run 1) and Inventive Examples 3, 5 and 7 when the inventive examples were combined with the conventional succinimide detergent. For example, Run 5 provided a greater power recovery than the arithmetic sum of the power recoveries provided by Runs 1 and 6 alone. Likewise Run 8 provided a power recovery that was greater than the arithmetic sum of the power recoveries of Runs 1 and 7.

For comparison purposes, the percent flow remaining was determined in the XUD-9 engine test as shown in Table 4. The XUD-9 test (CEC F-23-01 XUD-9 method) method is designed to evaluate the capability of a fuel to control the formation of deposits on the injector nozzles of an Indirect Injection diesel engine. All XUD-9 tests were run in DF-79 reference fuel. Results of tests run according to the XUD-9 test method are expressed in terms of the percentage airflow loss at various injector needle lift points. Airflow measurements are accomplished with an airflow rig complying with ISO 4010.

Prior to conducting the test, the injector nozzles are cleaned and checked for airflow at 0.05, 0.1, 0.2, 0.3 and 0.4 mm lift. Nozzles are discarded if the airflow is outside of the range 250 ml/min to 320 ml/min at 0.1 mm lift. The nozzles are assembled into the injector bodies and the opening pressures set to 115$\pm$5 bar. A slave set of injectors is also fitted to the engine. The previous test fuel is drained from the system. The engine is run for 25 minutes in order to flush through the fuel system. During this time all the spill-off fuel is discarded and not returned. The engine is then set to test speed and load and all specified parameters checked and adjusted to the test specification. The slave injectors are then replaced with the test units. Air flow is measured before and after the test. An average of 4 injector flows at 0.1 mm lift is used to calculate the percent of fouling. The degree of flow remaining = 100 - percent of fouling. The results are shown in the following table.

**Table 4**

| Fuel Additive | Treat rate (ppm by weight) | 0.1mm Lift Flow remaining (%) |
|---|---|---|
| Base fuel | NA | 23 |
| Conventional succinimide detergent (as described above in Table 3) | 50 | 33 |
| Base Fuel plus additive of Inventive Ex. 2 | 50 | 92 |
| Base Fuel plus additive of Example 9 | 50 | 39 |

The foregoing Table 4 illustrates the superior performance of Inventive Examples 2 and 9 in controlling the formation of deposits on fuel injectors compared to the base fuel devoid of the additive.

In the following example, the copper leachability of the additive was determined by aging copper coupons in ultra low sulfur fuel containing 10 wt.% of fatty methyl ester according to ASTM D-130. The additive treat rate in the fuel was 20 wt.% in order to accelerate the aging process. The amount of copper residue in the fuel was determined for each sample and is given in the following table.

**Table 5**

| Fuel Additive | Treat rate (weight %) | Copper (ppmw) |
|---|---|---|
| Base fuel | NA | 1 |

(continued)

| Fuel Additive | Treat rate (weight %) | Copper (ppmw) |
|---|---|---|
| Base Fuel plus additive of Carbonate Ex. 3 | 20 | 4 |
| Base Fuel plus additive of Inventive Ex. 7 | 20 | 1 |

As shown by the foregoing Table 5, Inventive Example 7 provided the same level of copper leachability as the base fuel devoid of any additive.

A demulsibility test according to ASTM D-1094 was conducted on several samples in order to determine the impact on fuel demulsibility of the reaction products in a fuel. The fuel used for the test was an ultra low sulfur diesel (ULSD) fuel having a pH buffered at 7 and including the additive at a treat rate of 200 ppmw. The fuel also contained 10 ppmw of a commercial polyglycol demulsifier. The results are shown in the following table.

**Table 6**

| Base ULSD fuel + Additive | Full Water Recovery Time | 1b Time | Separation at 5 minutes | Fuel clarity at 5 minutes |
|---|---|---|---|---|
| Carbonate Ex. 1 | Not achieved | Not achieved | 1 | 1 |
| Carbonate Ex. 2 | Not achieved | Not achieved | 3 | 4 |
| Carbonate Ex. 3 | Not achieved | Not achieved | 3 | 5 |
| Inventive Ex. 2 | 11 min. 15 sec. | 11 min. 30 sec. | 1 | 1 |
| Inventive Ex. 6 | 7 minutes | 13 min 30 sec. | 1 | 1 |

Table 6 illustrated that Inventive Examples 2 and 6 were effective, not only as detergents, but also exhibited improved demulsibility compared to the quaternary ammonium carbonate compounds of Carbonate Examples 1-3.

In the following example, a friction test was conducted using a high frequency reciprocating rig (HFRR) under a 200 gram load with a stroke distance of 1 millimeter at 50 Hz according to diesel fuel test ASTM D6079 except that the test was conducted in gasoline fuel at 25°C. The base fuel contained no additives. Each of the other fuel compositions contained a typical commercial Mannich base detergent package at 280 ppmw plus the additive being tested. The treat rate of the additive and the results are given in the following table.

**Table 7 - Fuel HFRR data**

| No. | Additive | Additive Treat rate (ppmw) | HFRR Wear (micrometer) Fuel 2 |
|---|---|---|---|
| 1 | Base fuel (no additives) | 0 | 750 |
| 2 | Base fuel plus Mannich base detergent package at 280 ppmw | 0 | 755 |
| 3 | No. 2 plus a propoxylated coco-diethanolamide friction modifier | 152 | 685 |
| 4 | No. 2 plus reaction product of isostearic acid and diethanolamine | 152 | 740 |
| 5 | No. 2 plus additive of Inventive Ex 3 | 152 | 535 |

The foregoing results showed the unexpected and superior wear protection provided by Inventive Example 3 compared to fuels containing conventional amide friction modifier in a fully formulated fuel composition.

An engine test measuring fuel injector deposit (referred to as "DIG test") was performed following a procedure

disclosed in Society of Automotive Engineer (SAE) International publication 2009-01-2641 "Test and Control of Fuel Injector Deposits in Direct Injected Spark Ignition Vehicles". A mathematical value of Long Term Fuel Trim (LTFT) was used to gauge the ability of additive to keep deposit from accumulating in the injectors, or to keep injectors clean. The higher the LTFT, the more deposit in the injectors and the less effective is the additive in keeping injectors clean.

The test may also be used to gauge the effectiveness of additives to clean up the injectors in a gasoline engine by running a standard 48 hour dirty up phase followed by a 48 hour clean up phase.

For the DIG test, a 2012 KIA OPTIMA equipped with a DISI 2.0 liter turbocharged 1-4 engine was used. The results are shown in the following table.

**Table 8**

| Run No. | Additives and treat rate (ppm by weight) | Normalized LTFT % |
|---|---|---|
| 1 | Gasoline with typical Mannich detergent[1] (81 ppmw) | 7 |
| 2 | Fuel and additive of Run 1 plus 12 ppmw of Inventive Example 3 | <1 |
| [1]Reaction product of dibutylamine, polyisobutylene cresol (1000 $MW_n$) and formaldehyde as generally described in US Patent No. 7,491,248. | | |

Table 8 illustrated that Inventive Example 3 also provided superior injector clean up properties in a gasoline fuel composition compared to a fuel containing only a conventional Mannich detergent.

It is noted that, as used in this specification and the appended claims, the singular forms "a," "an," and "the," include plural referents unless expressly and unequivocally limited to one referent. Thus, for example, reference to "an antioxidant" includes two or more different antioxidants. As used herein, the term "include" and its grammatical variants are intended to be non-limiting, such that recitation of items in a list is not to the exclusion of other like items that can be substituted or added to the listed items

Each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

## Claims

1. A fuel additive composition for a fuel injected engine comprising a fuel additive component and a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimeth-yl-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid wherein the quaternary ammonium carbonate is formed by reacting a carbonic acid diester with a tertiary amido amine compound of the following formula

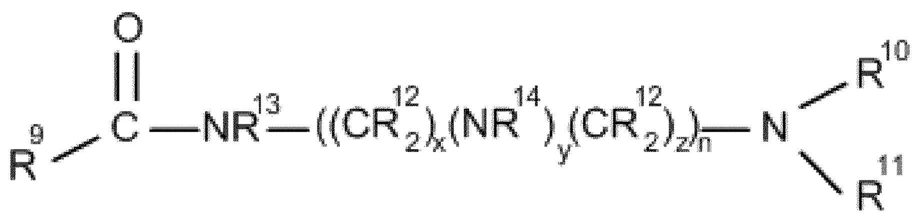

wherein each of $R^{10}$, and $R^{11}$ is selected from hydrocarbyl groups containing from 1 to 200 carbon atoms, each $R^9$, $R^{12}$, $R^{13}$ and $R^{14}$ is independently selected from hydrogen or a hydrocarbyl group, x is 1 to 6, y is 0 or 1, z is 1 to 6, and n is 1 to 6 or wherein the quaternary ammonium carbonate is selected from succinimidoalkyl trialkyl ammonium

carbonates, succinamido/succinyl ester ammonium carbonates and amidoalkyl trialkyl ammonium carbonates.

**2.** The fuel additive composition of claim 1, wherein from 0.5 to 2.0 equivalents of acid are reacted per equivalent of quaternary ammonium carbonate.

**3.** The fuel additive composition of any one of claims 1-2, wherein the fuel additive component is selected from a carrier fluid, a cetane improver, an octane improver, a fuel detergent, a demulsifier, an antioxidant, and a combination of two or more of the foregoing.

**4.** A fuel composition comprising the fuel additive of any one of claims 1-3, wherein the amount of hydrocarbyl soluble quaternary ammonium carboxylate ranges from 5 to 300 ppm based on a total weight of the fuel composition.

**5.** A diesel fuel composition comprising the fuel additive of any one of claims 1-3, wherein the amount of hydrocarbyl soluble quaternary ammonium carboxylate ranges from 10 to 200 ppm based on a total weight of the fuel composition, and wherein the fuel composition exhibits injector cleaning attributes and full water recovery and an interface rating of 1b in a demulsibility test according to ASTM D-1094.

**6.** A method of improving the injector performance of a fuel injected engine comprising operating the engine on a fuel composition comprising a major amount of fuel and a fuel additive composition for a fuel injected engine comprising a fuel additive component and a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimethyl-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid, wherein the hydrocarbyl soluble quaternary ammonium carboxylate is in an amount of from 5 to 300 ppm by weight based on a total weight of the fuel composition.

**7.** A method of reducing wear in a fuel system of an engine comprising combusting in the engine a fuel composition comprising a major amount of fuel and a fuel additive composition for a fuel injected engine comprising a fuel additive component and a hydrocarbyl soluble quaternary ammonium carboxylate derived from a quaternary ammonium carbonate and an organic acid selected from stearic acid, nonadecanoic acid, arachidic acid, tuberculostearic acid, petroselinic acid, oleic acid, elaidic acid, vaccenic acid, gadoleic acid, polyalkyl or polyalkenyl succinic ester acid, polyalkyl or polyalkenyl succinic amide acid, polyalkyl or polyalkenyl succinic imide acid, hexadecane diacid, heptadecane diacid, octadecane diacid, nonadecane diacid, eicosane diacid, 3-hexyl-4-decene-1,2-dicarboxylic acid, 3-hexyl-1,12-decanedicarboxylic acid, 6-ethylene-9-hexadecene-1,16-dicarboxylic acid, 6-ethyl-1,16-hexadecanedicarboxylic acid, 6-phenyl-1,12-dodecanedicarboxylic acid, 7,12-dimethyl-7,1-octadecanediene-1,18-dicarboxylic acid, 7,12-dimeth-yl-1,18-octadecanedicarboxylic acid, 6,8-diphenyl-1,14-tetradecanedicarboxylic acid, and polyalkyl or polyalkenyl succinic diacid, wherein the hydrocarbyl soluble quaternary ammonium carboxylate is in an amount of from 5 to 300 ppm by weight based on a total weight of the fuel composition.

**8.** The method of any one of claims 6-7, wherein the quaternary ammonium carbonate is selected from tetra-alkyl ammonium carbonates, succinimidoalkyl trialkyl ammonium carbonates, succinamido/succinyl ester ammonium carbonates, amidoalkyl trialkyl ammonium carbonates, hydrocarbyl ether trialkyl ammonium carbonate, and mixtures of trialkyl ammonium carbonates and tetra-alkyl ammonium carbonates.

**9.** The method of any one of claims 6-8, wherein from 0.5 to 2.0 equivalents of acid are reacted per equivalent of quaternary ammonium carbonate.

**10.** The method of any one of claims 6-9, wherein the fuel additive component is selected from a carrier fluid, a cetane improver, an octane improver, a fuel detergent, a demulsifier, an antioxidant, and a combination of two or more of the foregoing.

**11.** The method of any one of claim 6 and claims 7-10 when dependent on claim 6, wherein the engine is selected from the group consisting of a direct fuel injected diesel engine and a direct fuel injected gasoline engine.

## Patentansprüche

1. Kraftstoffzusatzstoffzusammensetzung für einen Kraftstoffeinspritzmotor, umfassend einen Kraftstoffzusatzstoffbestandteil und ein lösliches quartäres Hydrocarbylammoniumcarboxylat, abgeleitet von einem quartären Ammoniumcarbonat und einer organischen Säure, ausgewählt aus Stearinsäure, Nonadecansäure, Arachinsäure, Tuberculostearinsäure, Petroselinsäure, Oleinsäure, Elaidinsäure, Vaccensäure, Gadoleinsäure, Polyalkyl- oder Polyalkenylbernsteinestersäure, Polyalkyl- oder Polyalkenylsuccinamidsäure, Polyalkyl- oder Polyalkenylsuccinimidsäure, Hexadecandisäure, Heptadecandisäure, Octadecandisäure, Nonadecandisäure, Eicosandisäure, 3-Hexyl-4-decen-1,2-dicarbonsäure, 3-Hexyl-1,12-decandicarbonsäure, 6-Ethylen-9-hexadecen-1,16-dicarbonsäure, 6-Ethyl-1,16-hexadecandicarbonsäure, 6-Phenyl-1,12-dodecandicarbonsäure, 7,12-Dimethyl-7,1-octadecandien-1,18-dicarbonsäure, 7,12-Dimeth-yl-1,18-octadecandicarbonsäure, 6,8-Diphenyl-1,14-tetradecandicarbonsäure und Polyalkyl- oder Polyalkenylbernsteindisäure,
wobei das quartäre Ammoniumcarbonat durch Umsetzen eines Kohlensäurediesters mit einer tertiären Amidoaminverbindung der folgenden Formel gebildet wird

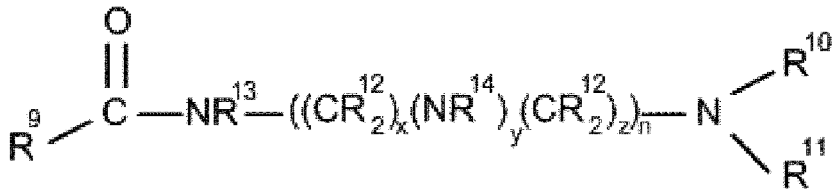

wobei jedes von $R^{10}$ und $R^{11}$ aus Hydrocarbylgruppen mit 1 bis 200 Kohlenstoffatomen ausgewählt ist, jedes $R^9$, $R^{12}$, $R^{13}$ und $R^{14}$ unabhängig voneinander aus Wasserstoff oder einer Hydrocarbylgruppe ausgewählt ist, x 1 bis 6 ist, y 0 oder 1 ist, z 1 bis 6 ist und n 1 bis 6 ist oder wobei das quartäre Ammoniumcarbonat aus Succinimidoalkyltrialkylammoniumcarbonaten, Succinamido-/Succinylesterammoniumcarbonaten und Amidoalkyltrialkylammoniumcarbonaten ausgewählt ist.

2. Kraftstoffzusatzstoffzusammensetzung nach Anspruch 1, wobei von 0,5 bis 2,0 Äquivalente von Säure pro Äquivalent von quartärem Ammoniumcarbonat umgesetzt werden.

3. Kraftstoffzusatzstoffzusammensetzung nach einem der Ansprüche 1-2, wobei der Kraftstoffzusatzstoffbestandteil aus einer Trägerflüssigkeit, einem Cetanverbesserer, einem Octanverbesserer, einem Kraftstoffreiniger, einem Demulgator, einem Antioxidationsmittel und einer Kombination von zwei oder mehr der Vorstehenden ausgewählt ist.

4. Kraftstoffzusammensetzung, umfassend den Kraftstoffzusatzstoff nach einem der Ansprüche 1-3, wobei die Menge von löslichem quartären Hydrocarbylammoniumcarboxylat im Bereich von 5 bis 300 ppm, bezogen auf ein Gesamtgewicht der Kraftstoffzusammensetzung, liegt.

5. Dieselkraftstoffzusammensetzung, umfassend den Kraftstoffzusatzstoff nach einem der Ansprüche 1-3, wobei die Menge von löslichem quartären Hydrocarbylammoniumcarboxylat im Bereich von 10 bis 200 ppm, bezogen auf ein Gesamtgewicht der Kraftstoffzusammensetzung, liegt und wobei die Kraftstoffzusammensetzung Einspritzerreinigungsattribute und vollständige Wasserrückgewinnung und eine Grenzflächenbewertung von Ib in einem Demulgiervermögenstest gemäß ASTM D-1094 aufweist.

6. Verfahren zum Verbessern der Einspritzleistung eines Kraftstoffeinspritzmotors, umfassend das Betreiben des Motors mit einer Kraftstoffzusammensetzung, umfassend eine Hauptmenge von Kraftstoff und eine Kraftstoffzusatzstoffzusammensetzung für einen Kraftstoffeinspritzmotor, umfassend einen Kraftstoffzusatzstoffbestandteil und ein lösliches quartäres Hydrocarbylammoniumcarboxylat, abgeleitet von einem quartären Ammoniumcarbonat und einer organischen Säure, ausgewählt aus Stearinsäure, Nonadecansäure, Arachinsäure, Tuberculostearinsäure, Petroselinsäure, Oleinsäure, Elaidinsäure, Vaccensäure, Gadoleinsäure, Polyalkyl- oder Polyalkenylbernsteinestersäure, Polyalkyl- oder Polyalkenylsuccinamidsäure, Polyalkyl- oder Polyalkenylsuccinimidsäure, Hexadecandisäure, Heptadecandisäure, Octadecandisäure, Nonadecandisäure, Eicosandisäure, 3-Hexyl-4-decen-1,2-dicarbonsäure, 3-Hexyl-1,12-decandicarbonsäure, 6-Ethylen-9-hexadecen-1,16-dicarbonsäure, 6-Ethyl-1,16-hexadecandicarbonsäure, 6-Phenyl-1,12-dodecandicarbonsäure, 7,12-Dimethyl-7,1 -octadecandien-1,18-dicarbonsäure, 7,12-Dimeth-yl-1,18-octadecandicarbonsäure, 6,8-Diphenyl-1,14-tetradecandicarbonsäure und Polyalkyl- oder Polyalkenylbernsteindisäure, wobei das lösliche quartäre Hydrocarbylammoniumcarboxylat in einer Menge von 5 bis 300

ppm, bezogen auf ein Gesamtgewicht der Kraftstoffzusammensetzung, vorliegt.

7. Verfahren zum Reduzieren von Verschleiß in einem Kraftstoffsystem eines Motorn, umfassend das Verbrennen einer Kraftstoffzusammensetzung im Motor, umfassend eine Hauptmenge von Kraftstoff und eine Kraftstoffzusatzstoffzusammensetzung für einen Kraftstoffeinspritzmotor, umfassend einen Kraftstoffzusatzstoffbestandteil und ein lösliches quartäres Hydrocarbylammoniumcarboxylat, abgeleitet von einem quartären Ammoniumcarbonat und einer organischen Säure, ausgewählt aus Stearinsäure, Nonadecansäure, Arachinsäure, Tuberculostearinsäure, Petroselinsäure, Oleinsäure, Elaidinsäure, Vaccensäure, Gadoleinsäure, Polyalkyl- oder Polyalkenylbernsteinestersäure, Polyalkyl- oder Polyalkenylsuccinamidsäure, Polyalkyl- oder Polyalkenylsuccinimidsäure, Hexadecandisäure, Heptadecandisäure, Octadecandisäure, Nonadecandisäure, Eicosandisäure, 3-Hexyl-4-decen-1,2-dicarbonsäure, 3-Hexyl-1,12-decandicarbonsäure, 6-Ethylen-9-hexadecen-1,16-dicarbonsäure, 6-Ethyl-1,16-hexadecandicarbonsäure, 6-Phenyl-1,12-dodecandicarbonsäure, 7,12-Dimethyl-7,1 -octadecandien-1,18-dicarbonsäure, 7,12-Dimeth-yl-1,18-octadecandicarbonsäure, 6,8-Diphenyl-1,14-tetradecandicarbonsäure und Polyalkyl- oder Polyalkenylbernsteindisäure, wobei das lösliche quartäre Hydrocarbylammoniumcarboxylat in einer Menge von 5 bis 300 ppm, bezogen auf ein Gesamtgewicht der Kraftstoffzusammensetzung, vorliegt.

8. Verfahren nach einem der Ansprüche 6-7, wobei das quartäre Ammoniumcarbonat aus Tetraalkylammoniumcarbonaten, Succinimidoalkyltrialkylammoniumcarbonaten, Succinamido-/succinylesterammoniumcarbonaten, Amidoalkyltrialkylammoniumcarbonaten, Hydrocarbylethertrialkylammoniumcarbonat und Mischungen von Trialkylammoniumcarbonaten und Tetraalkylammoniumcarbonaten ausgewählt ist.

9. Verfahren nach einem der Ansprüche 6-8, wobei von 0,5 bis 2,0 Äquivalente von Säure pro Äquivalent von quartärem Ammoniumcarbonat umgesetzt werden.

10. Verfahren nach einem der Ansprüche 6-9, wobei der Kraftstoffzusatzstoffbestandteil aus einer Trägerflüssigkeit, einem Cetanverbesserer, einem Octanverbesserer, einem Kraftstoffreiniger, einem Demulgator, einem Antioxidationsmittel und einer Kombination von zwei oder mehr der Vorstehenden ausgewählt ist.

11. Verfahren nach einem der Ansprüche 6 und Ansprüche 7-10 in Abhängigkeit von Anspruch 6, wobei der Motor ausgewählt ist aus der Gruppe bestehend aus einem Dieselkraftstoffdirekteinspritzmotor und einem Benzinkraftstoffdirekteinspritzmotor.

## Revendications

1. Composition d’additif pour carburant destinée à un moteur à injection de carburant comprenant un composant d’additif pour carburant et un carboxylate d’ammonium quaternaire soluble hydrocarbyle dérivé d’un carbonate d’ammonium quaternaire et d’un acide organique choisi parmi l’acide stéarique, l’acide nonadécanoïque, l’acide arachidique, l’acide tuberculostéarique, l’acide pétrosélinique, l’acide oléique, l’acide élaïdique, l’acide vaccénique, l’acide gadoléique, l’acide d’ester succinique polyalkyle ou polyalcényle, l’acide d’amide succinique polyalkyle ou polyalcényle, l’acide d’imide succinique polyalkyle ou polyalcényle, le diacide d’hexadécane, le diacide d’heptadécane, le diacide d’octadécane, le diacide de nonadécane, le diacide d’eicosane, l’acide 3-hexyl-4-décène-1, 2-dicarboxylique, l’acide 3-hexyl-1,12-décanedicarboxylique, l’acide 6-éthylène-9-hexadécène-1, 16-dicarboxylique, l’acide 6-éthyl-1,16-hexadécanedicarboxylique, l’acide 6-phényl-1,12-dodécanedicarboxylique, l’acide 7,12-diméth-yl-7,1-octadécanediène-1,18-dicarboxylique, l’acide 7,12-diméth-yl-1,18-octadécanedicarboxylique, l’acide 6,8-diphényl-1,14-tétradécanedicarboxylique, et le diacide succinique polyalkyle ou polyalcényle
dans laquelle le carbonate d’ammonium quaternaire est formé en faisant réagir un diester d’acide carbonique avec un composé d’amine amido tertiaire de la formule suivante :

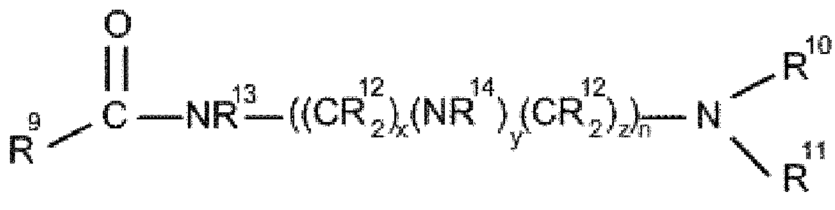

dans laquelle chacun de $R^{10}$, et $R^{11}$ est choisi parmi des groupes hydrocarbyle contenant de 1 à 200 atomes de

carbone, chaque $R^9$, $R^{12}$, $R^{13}$ et $R^{14}$ est indépendamment choisi parmi l'hydrogène ou un groupe hydrocarbyle, x va de 1 à 6, y est 0 ou 1, z va de 1 à 6, et n va de 1 à 6 ou dans laquelle le carbonate d'ammonium quaternaire est choisi parmi des carbonates d'ammonium trialkyle succinimidoalkyle, des carbonates d'ammonium d'ester succinamido/succinyle et des carbonates d'ammonium trialkyle amidoalkyle.

**2.** Composition d'additif pour carburant selon la revendication 1, dans laquelle de 0,5 à 2,0 équivalents d'acide sont mis en réaction par équivalent de carbonate d'ammonium quaternaire.

**3.** Composition d'additif pour carburant selon l'une quelconque des revendications 1 et 2, dans laquelle le composant d'additif pour carburant est choisi parmi un fluide véhicule, un agent améliorant de cétane, un agent améliorant d'octane, un détergent pour carburant, un agent désémulsifiant, un antioxydant et une combinaison de deux ou plus de ceux-ci.

**4.** Composition de carburant comprenant l'additif pour carburant selon l'une quelconque des revendications 1 à 3, dans laquelle la quantité de carboxylate d'ammonium quaternaire soluble hydrocarbyle va de 5 à 300 ppm sur la base d'un poids total de la composition de carburant.

**5.** Composition de carburant diesel comprenant l'additif pour carburant selon l'une quelconque des revendications 1 à 3, dans laquelle la quantité de carboxylate d'ammonium quaternaire soluble hydrocarbyle varie de 10 à 200 ppm sur la base d'un poids total de la composition de carburant, et dans laquelle la composition de carburant présente des attributs de nettoyage d'injecteur et une récupération d'eau totale et une évaluation d'interface de 1b dans un test de démulsibilité selon ASTM D-1094.

**6.** Procédé d'amélioration de la performance d'un injecteur d'un moteur à injection de carburant comprenant le fonctionnement du moteur sur une composition de carburant comprenant une quantité majeure de carburant et une composition d'additif pour carburant pour un moteur à injection de carburant comprenant un composant d'additif pour carburant et un carboxylate d'ammonium quaternaire soluble hydrocarbyle dérivé d'un carbonate d'ammonium quaternaire et d'un acide organique choisi parmi l'acide stéarique, l'acide nonadécanoïque, l'acide arachidique, l'acide tuberculostéarique, l'acide pétrosélinique, l'acide oléique, l'acide élaïdique, l'acide vaccénique, l'acide gadoléique, l'acide d'ester succinique polyalkyle ou polyalcényle, l'acide d'amide succinique polyalkyle ou polyalcényle, l'acide d'imide succinique polyalkyle ou polyalcényle, le diacide hexadécane, le diacide heptadécane, le diacide octadécane, le diacide nonadécane, le diacide eicosane, l'acide 3-hexyl-4-décène-1,2-dicarboxylique, l'acide 3-hexyl-1,12-décanedicarboxylique, l'acide 6-éthylène-9-hexadécène-1,16-dicarboxylique, acide 6-éthyl-1,16-hexadécanedicarboxylique, l'acide 6-phényl-1,12-dodécanedicarboxylique, l'acide 7,12-diméth-yl-7,1-octadécanediène-1,18-dicarboxylique, l'acide 7,12-diméth-yl-1,18-octadécanedicarboxylique, l'acide 6,8-diphényl-1,14-tétradécanedicarboxylique, et le diacide succinique polyalkyle ou polyalcényle, dans lequel le carboxylate d'ammonium quaternaire soluble hydrocarbyle est dans une quantité allant de 5 à 300 ppm en poids sur la base d'un poids total de la composition de carburant.

**7.** Procédé de réduction d'usure dans un système de carburant d'un moteur comprenant la combustion dans le moteur d'une composition de carburant comprenant une quantité majeure de carburant et une composition d'additif pour carburant pour un moteur à injection de carburant comprenant un composant d'additif pour carburant et un carboxylate d'ammonium quaternaire soluble hydrocarbyle dérivé d'un carbonate d'ammonium quaternaire et d'un acide organique choisi parmi l'acide stéarique, l'acide nonadécanoïque, l'acide arachidique, l'acide tuberculostéarique, l'acide pétrosélinique, l'acide oléique, l'acide élaïdique, l'acide vaccénique, l'acide gadoléique, l'acide d'ester succinique polyalkyle ou polyalcényle, l'acide d'amide succinique polyalkyle ou polyalcényle, l'acide d'imide succinique polyalkyle ou polyalcényle, le diacide d'hexadécane, le diacide d'heptadécane, le diacide d'octadécane, le diacide de nonadécane, le diacide d'eicosane, l'acide 3-hexyl-4-décène-1,2-dicarboxylique, l'acide 3-hexyl-1,12-décanedicarboxylique, l'acide 6-éthylène-9-hexadécène-1,16-dicarboxylique, l'acide 6-éthyl-1,16-hexadécanedicarboxylique, l'acide 6-phényl-1,12-dodécanedicarboxylique, l'acide 7,12-diméth-yl-7,1-octadécanediène-1,18-dicarboxylique, l'acide 7,12-diméth-yl-1,18-octadécanedicarboxylique, l'acide 6,8-diphényl-1,14-tétradécanedicarboxylique, et le diacide succinique polyalkyle ou polyalcényle, dans lequel le carboxylate d'ammonium quaternaire soluble hydrocarbyle est dans une quantité allant de 5 à 300 ppm en poids sur la base d'un poids total de la composition de carburant.

**8.** Procédé selon l'une quelconque des revendications 6 à 7, dans lequel le carbonate d'ammonium quaternaire est choisi parmi les carbonates d'ammonium tétra-alkyle, les carbonates d'ammonium trialkyle succinimidoalkyle, les carbonates d'ammonium d'ester succinamido/succinyle, les carbonates d'ammonium trialkyle amidoalkyle, le carbonate d'ammonium trialkyle d'éther hydrocarbyle, et les mélanges de carbonates d'ammonium trialkyle et de carbonates d'ammonium de tétra-alkyle.

**9.** Procédé selon l'une quelconque des revendications 6 à 8, dans lequel de 0,5 à 2,0 équivalents d'acide sont mis en réaction par équivalent de carbonate d'ammonium quaternaire.

**10.** Procédé selon l'une quelconque des revendications 6 à 9, dans lequel le composant additif pour carburant est choisi parmi un fluide porteur, un agent améliorant de cétane, un agent améliorant d'octane, un détergent de carburant, un désémulsifiant, un antioxydant et une combinaison de deux ou plus de ceux-ci.

**11.** Procédé selon l'une quelconque de la revendication 6 et des revendications 7 à 10 lorsqu'elles dépendent de la revendication 6, dans lequel le moteur est choisi dans le groupe constitué d'un moteur diesel à injection directe de carburant et d'un moteur à essence à injection directe de carburant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8147569 B
- EP 0293192 A1
- WO 2013070503 A1
- US 8475541 B
- WO 2009012641 A
- US 7491248 B